# EUROPEAN PATENT APPLICATION

(11) **EP 4 643 919 A1**
(43) Date of publication of application: **05.11.2025**
(21) Application number: 24741586.2
(22) Date of filing: 12.01.2024
(51) Int. Cl.: A61M 25/06, A61M 5/158

(54) **PUNCTURE NEEDLE**

(30) Priority: 13.01.2023 JP 2023003575
(71) Applicant: Terumo Kabushiki Kaisha, Tokyo 151-0072 (JP)
(72) Inventor: MAEDA, Ryosuke, Fujinomiya-shi, Shizuoka 418-0015 (JP); WADA, Satoshi, Fujinomiya-shi, Shizuoka 418-0015 (JP)
(74) Representative: Casalonga
(86) International application number: PCT/JP2024/000568
(87) International publication number: WO 2024/150814

(57) **Abstract**

Provided is a puncture needle enabled to smoothly perform puncture without being affected by hardening of a biological organ to be punctured, or the like.

A puncture needle 10 includes an outer needle 100, and an inner needle 200 configured to be insertable into the outer needle, in which in a connected state in which an inner needle main body 210 of the inner needle is inserted into an outer needle main body 110 of the outer needle 100 and an inner needle hub 270 and an outer needle hub 170 are connected together, a most distal end 210b of an inner needle distal end 210a is located on the distal end side from a most distal end 135 of a first protruding portion 130 of the outer needle and a most distal end 145 of a second protruding portion 140 of the outer needle, and the inner needle distal end of the inner needle is disposed such that an inclined surface 220 forms a gap g1 between an inner surface 133 of the first protruding portion and/or an inner surface 143 of the second protruding portion.

## Description

### Technical Field

The present invention relates to a puncture needle.

### Background Art

Conventionally, in the medical field, as a puncture needle for puncturing a skin, a blood vessel, an organ, or the like of a patient, there are ones for various applications. Examples of the puncture needle include an injection needle, a blood sampling needle, and an indwelling needle. Patent Literature 1 discloses a puncture needle (introducer needle) for forming an access path connecting the outside of a patient's living body to the inside of a blood vessel before delivering a catheter into the living body.

### Citation List

### Patent Literature

Patent Literature 1: JP 2021-049233 A

### Summary of Invention

### Technical Problem

In a procedure using the puncture needle described in Patent Literature 1, in a state in which an outer needle and an inner needle inserted inside the outer needle are assembled together, the blood vessel is punctured with the distal end side of each needle. After performing puncture with the puncture needle, an operator removes the inner needle from the outer needle, and introduces a medical instrument such as a guide wire and a medical device such as a catheter into the blood vessel through the inner cavity of the outer needle, or directly introduce the medical device without using the guide wire or the like in some cases.

In the procedure using the puncture needle including the outer needle and the inner needle as described above, smooth progress of the procedure may be hindered depending on a state of the blood vessel to be punctured.

For example, in a case where a blood vessel to be treated is hardened and stenosed due to progress of plaque or the like, the operator may perform a procedure of introducing a penetrating wire or the like having a small diameter and high rigidity into the stenosed part in order to treat the stenosed part of the blood vessel to be treated. Before introducing the penetrating wire into the blood vessel, the operator uses the puncture needle including the outer needle and the inner needle as described above to form a perforation in a blood vessel as a puncture target site separated from the blood vessel to be treated. At this time, since a thin blood vessel located on the peripheral side of the blood vessel to be treated is selected as the puncture target site, the operator has to select a puncture needle having a small diameter in accordance with the blood vessel having a small diameter. It is difficult to ensure sufficient rigidity for the puncture needle with a reduced diameter, and it is difficult to smoothly perform puncture in a case where the blood vessel of the puncture target site has hardened (for example, in a case where a calcified lesion is formed in which arteriosclerosis has progressed). Note that it is considered that the problem as described above can occur at the time of treatment of various lesions and the like generated in various sites of a biological organ even in a case other than a blood vessel to be treated in which a stenosed part is formed.

An object of the present invention is to provide a puncture needle enabled to smoothly perform puncture without being affected by hardening of a biological organ to be punctured, or the like.

### Solution to Problem

(1) A puncture needle including an outer needle, and an inner needle configured to be insertable into the outer needle, in which: the outer needle includes an outer needle main body having an inner cavity, and an outer needle hub disposed on a proximal end side of the outer needle main body; the inner needle includes an inner needle main body having an inner needle distal end that is sharp, and an inner needle hub connected to the proximal end side of the inner needle main body; a distal end of the outer needle main body includes a first protruding portion and a second protruding portion each having a blade surface on a side surface; the inner needle distal end includes an inclined surface; and in a connected state in which the inner needle main body is inserted into the outer needle main body and the inner needle hub and the outer needle hub are connected together, a most distal end of the inner needle distal end is located on a distal end side from a most distal end of the first protruding portion and a most distal end of the second protruding portion, and the inner needle distal end is disposed such that the inclined surface forms a gap with an inner surface of the first protruding portion and/or an inner surface of the second protruding portion.
(2) The puncture needle according to (1), in which the first protruding portion and the second protruding portion are disposed at equal intervals in a circumferential direction along a virtual circle centered on the most distal end of the inner needle distal end in the connected state.
(3) The puncture needle according to (1) or (2), in which the most distal end of the first protruding portion and the most distal end of the second protruding portion are disposed on the distal end side from a proximal end of the inclined surface in the connected state.
(4) The puncture needle according to any one of (1) to (3), in which a certain range on the proximal end side from the most distal end of the first protruding portion and a certain range on the proximal end side from the most distal end of the second protruding portion are inclined toward an axial center side of the outer needle main body toward the distal end side from the proximal end side of the outer needle main body.
(5) The puncture needle according to any one of (1) to (4), in which hardness of the outer needle main body is larger than hardness of the inner needle main body.
(6) The puncture needle according to any one of (1) to (5), in which a recessed groove portion extending along an axial direction of the inner needle main body is formed on an outer surface of the inner needle main body.
(7) The puncture needle according to any one of (1) to (6), in which: the inner needle main body of the inner needle has a solid structure; the outer needle has a hollow structure in which the inner cavity of the outer needle main body is gradually larger at a position closer to the proximal end side from the distal end side; and a gap portion through which liquid is allowed to flow is formed between an outer surface of the inner needle main body and an inner surface of the outer needle main body in the connected state.
(8) The puncture needle according to any one of (1) to (7), in which: the outer needle includes a first uneven portion that enhances echo visibility and is formed in a predetermined range toward the proximal end side from the most distal end of the first protruding portion and the most distal end of the second protruding portion; and the inner needle includes a second uneven portion that enhances echo visibility and is formed between the most distal end of the inner needle distal end and a proximal end of the inclined surface.

### Advantageous Effects of Invention

In the connected state in which the inner needle and the outer needle are connected together, the puncture needle of (1) is disposed in a state in which the most distal end of the inner needle distal end and the most distal end of each protruding portion of the outer needle are exposed at the distal end of the puncture needle. In addition, in the connected state, the most distal end of the inner needle distal end is located on the distal end side from the most distal end of the first protruding portion of the outer needle and the most distal end of the second protruding portion of the outer needle. When puncturing the puncture target site with the puncture needle, the operator rotates the puncture needle with the most distal end of the inner needle as a fulcrum. When the operator rotates the puncture needle, the most distal end of each protruding portion of the outer needle starts to hit the puncture target site in a state in which the most distal end of the inner needle distal end is in contact with the puncture target site. The operator rotates the puncture needle to rotate each protruding portion of the outer needle located around the inner needle, thereby being able to form the perforation in such a manner as to crush the puncture target site by each protruding portion of the outer needle. For that reason, for example, even in a case where the puncture target site includes a calcified lesion or the like that has hardened, the operator can smoothly form the perforation with the puncture needle. In addition, in the puncture needle in the connected state, the inclined surface of the inner needle distal end is disposed to form a gap with the inner surface of the first protruding portion of the outer needle and/or the inner surface of the second protruding portion of the outer needle. For that reason, when an operation is performed of rotating the puncture needle with the most distal end of the inner needle distal end as a fulcrum, it is possible to move the outer needle and the inner needle toward a puncture direction while releasing a part of the puncture target site (for example, a part of a biological tissue or a part of a calcified lesion) into the gap. For that reason, the operator can smoothly form the perforation toward a predetermined direction by rotating the puncture needle while bringing the most distal end of each protruding portion and the most distal end of the inner needle distal end into contact with the puncture target site.

### Brief Description of Drawings

Fig. 1 is a diagram illustrating a puncture needle according to a first embodiment, and is a plan view of a state before an inner needle and an outer needle are connected together.
Fig. 2 is a diagram illustrating the puncture needle according to the first embodiment, and is a plan view of a state in which the inner needle and the outer needle are connected together.
Fig. 3 is a front view of an inner needle main body viewed from a distal end side.
Fig. 4 is a perspective view of an inner needle distal end viewed from a specific direction.
Fig. 5 is a perspective view of the inner needle distal end viewed from a direction different from the direction illustrated in Fig. 4.
Fig. 6 is a plan view of the distal end of an outer needle main body.
Fig. 7 is a perspective view of the distal end of the outer needle main body viewed from a specific direction.
Fig. 8 is a perspective view of the distal end of the outer needle main body viewed from a direction different from the direction illustrated in Fig. 7.
Fig. 9 is a perspective view of the distal end of the puncture needle in a connected state viewed from a specific direction.
Fig. 10 is a perspective view of the distal end of the puncture needle in the connected state viewed from a direction different from the direction illustrated in Fig. 9.
Fig. 11 is a diagram illustrating an outer needle hub and an inner needle hub in the connected state in an enlarged manner.
Fig. 12A is a diagram illustrating an arrangement example of the inner needle and the outer needle in the connected state in a simplified manner.
Fig. 12B is a diagram illustrating an arrangement example of the inner needle and the outer needle in the connected state in a simplified manner.
Fig. 13A is a diagram illustrating an arrangement example of the inner needle and the outer needle in the connected state in a simplified manner.
Fig. 13B is a diagram illustrating an arrangement example of the inner needle and the outer needle in the connected state in a simplified manner.
Fig. 14A is a diagram illustrating an arrangement example of the inner needle and the outer needle in the connected state in a simplified manner.
Fig. 14B is a diagram illustrating an arrangement example of the inner needle and the outer needle in the connected state in a simplified manner.
Fig. 15A is a diagram illustrating an arrangement example of the inner needle and the outer needle in the connected state in a simplified manner.
Fig. 15B is a diagram illustrating an arrangement example of the inner needle and the outer needle in the connected state in a simplified manner.
Fig. 16 is a diagram for explaining action of the puncture needle.
Fig. 17 is a diagram for explaining the action of the puncture needle.
Fig. 18 is a diagram illustrating a positional relationship between the inner needle and the outer needle when the puncture needle is used.
Fig. 19 is a cross-sectional view schematically illustrating a usage example of the puncture needle.
Fig. 20 is a cross-sectional view schematically illustrating the usage example of the puncture needle.
Fig. 21 is a perspective view of an inner needle according to Modification 1.
Fig. 22 is a perspective view of an outer needle according to Modification 2 viewed from a specific direction.
Fig. 23 is a perspective view of the outer needle according to Modification 2 viewed from a direction different from the direction illustrated in Fig. 22.
Fig. 24 is a perspective view of an inner needle according to Modification 3.
Fig. 25 is a diagram for explaining a puncture needle according to Modification 4, and is a diagram illustrating a positional relationship between the inner needle and the outer needle when the puncture needle is used.
Fig. 26 is a diagram for explaining a puncture needle according to Modification 5, and is a diagram illustrating a positional relationship between the inner needle and the outer needle when the puncture needle is used.

### Description of Embodiments

Hereinafter, embodiments of the present invention will be described with reference to the accompanying drawings. The following description does not limit the technical scope or the significance of each term disclosed in the claims. Furthermore, dimensional ratios of the drawings are exaggerated for illustration purpose and may differ from actual ratios.

### (First Embodiment)

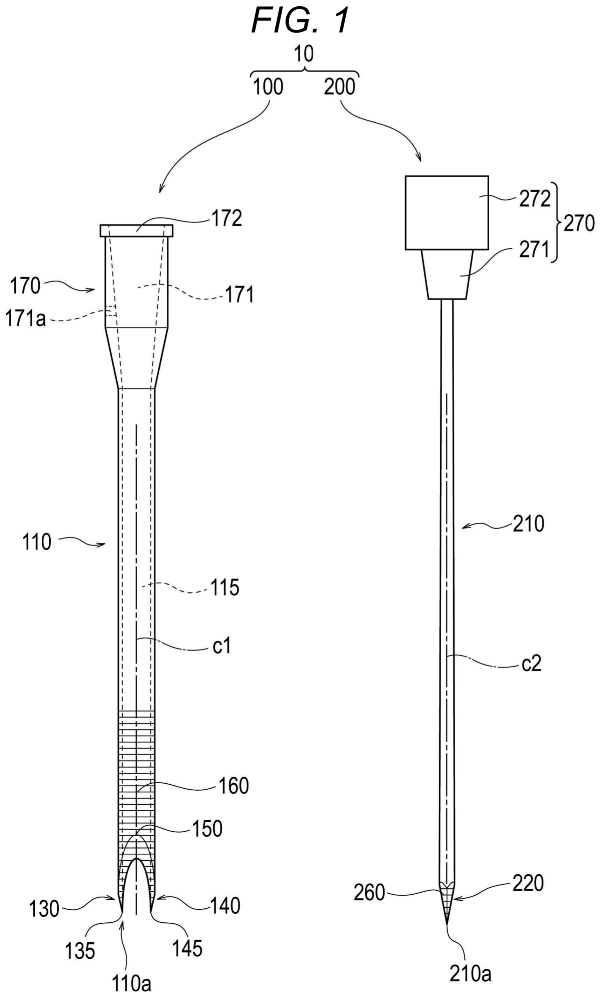

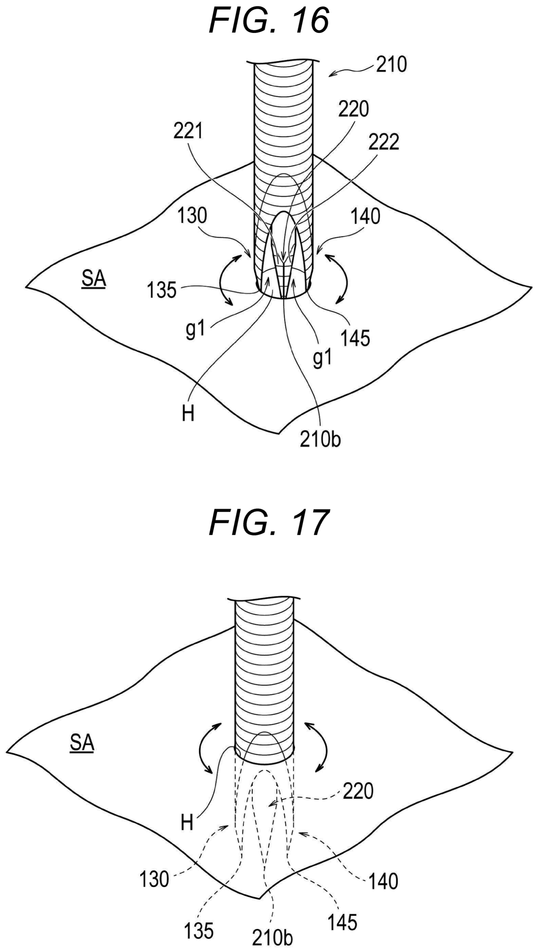

Figs. 1 to 15B are diagrams for explaining a puncture needle 10 according to the present embodiment. Figs. 16 to 20 are diagrams for explaining action and effect and a usage example of the puncture needle 10.

### <Puncture Needle 10>

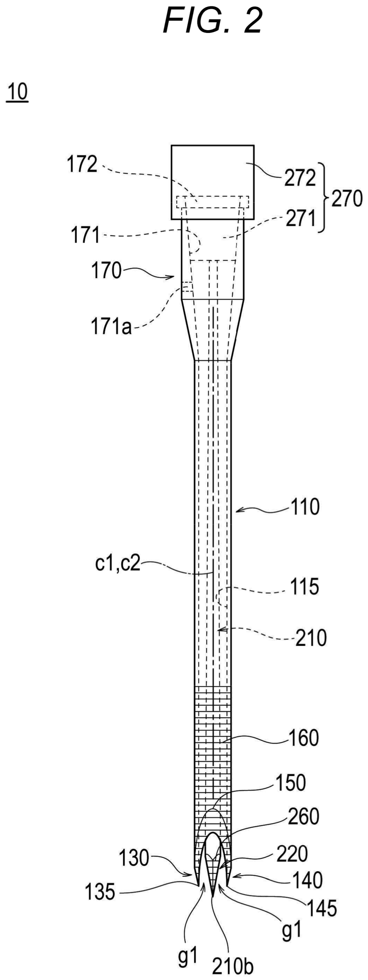

The puncture needle 10 generally includes an outer needle 100, and an inner needle 200 configured to be insertable into the outer needle 100 as illustrated in Figs. 1 and 2.

An operator can use the puncture needle 10 in a procedure for forming a predetermined perforation H in a connected state in which an inner needle main body 210 is inserted into an outer needle main body 110, and an inner needle hub 270 and an outer needle hub 170 are connected together (A state illustrated in Fig. 2. The state is also simply referred to as "connected state" in the present specification).

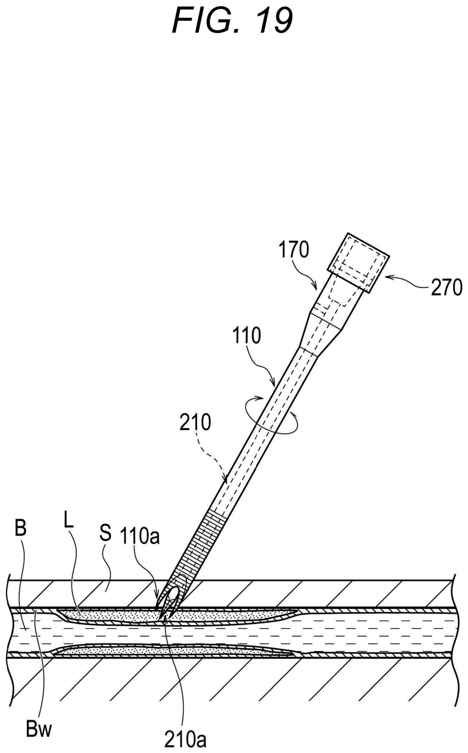

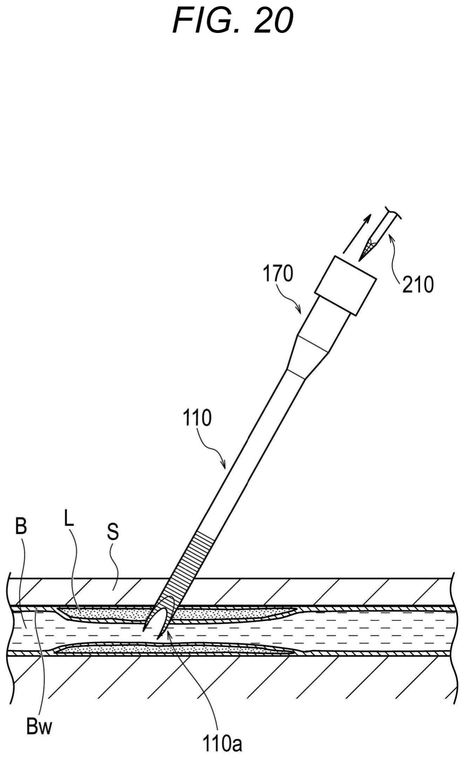

For example, as illustrated in Figs. 19 and 20, the puncture needle 10 can be configured as a medical instrument for performing puncture on a blood vessel B such as an artery. In the present embodiment, a description will be given of an example in which the puncture needle 10 is configured as an indwelling needle (introduction needle) for puncturing a blood vessel wall Bw of the blood vessel B in which a calcified lesion L is formed, skin tissue S between the blood vessel wall Bw and the outside of a living body, and the calcified lesion L.

Note that the specific application of the puncture needle 10 and the biological organ to be applied are not particularly limited. The puncture needle 10 can also be applied to a medical instrument having a needle structure known in the medical field, such as an injection needle, a blood sampling needle, and a biopsy needle.

### <Outer Needle 100>

As illustrated in Figs. 1 and 2, the outer needle 100 includes the outer needle main body 110 having an inner cavity 115, and the outer needle hub 170 disposed on the proximal end side of the outer needle main body 110.

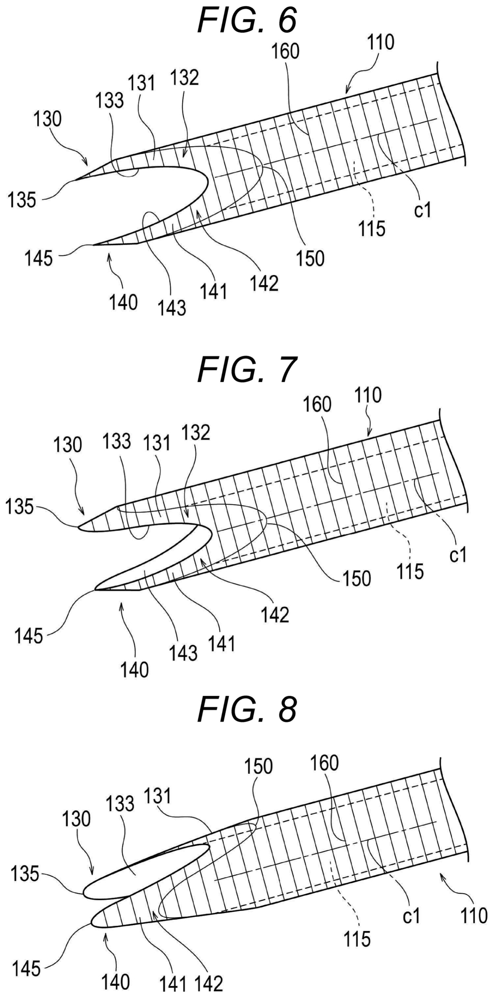

As illustrated in Figs. 6, 7, and 8, a distal end 110a of the outer needle main body 110 includes a first protruding portion 130 and a second protruding portion 140.

The "distal end (or distal end side)" in the present specification means a side (lower side in Figs. 1 and 2) on which the puncture needle 10 is inserted into the living body, and the "proximal end (or proximal end side)" means a side (upper side in Figs. 1 and 2) located opposite to the distal end.

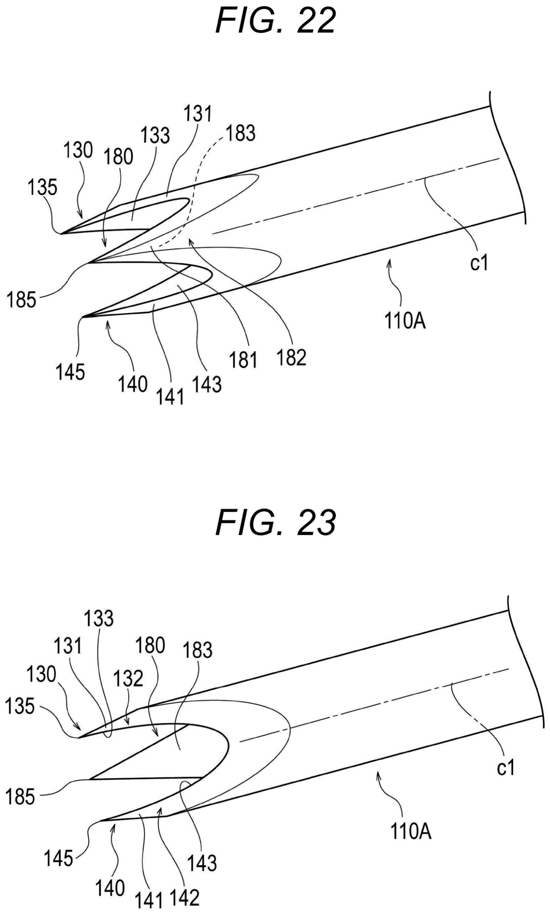

As illustrated in Figs. 6, 7, and 8, the first protruding portion 130 includes a blade surface 132 on a side surface 131 thereof. The second protruding portion 140 includes a blade surface 142 on a side surface 141 thereof. Note that, in the present embodiment, the outer needle 100 including the two protruding portions 130 and 140 is exemplified, but the number of protruding portions included in the outer needle 100 only needs to be two or more. In Modification 2 described later, an outer needle including three protruding portions 130, 140, and 180 is exemplified (see Figs. 22 and 23).

Between the first protruding portion 130 and the second protruding portion 140, a space is provided in which a most distal end 210b of an inner needle distal end 210a described later can be disposed. The space is located on the distal end side of the inner cavity 115 of the outer needle main body 110 and communicates with the inner cavity 115.

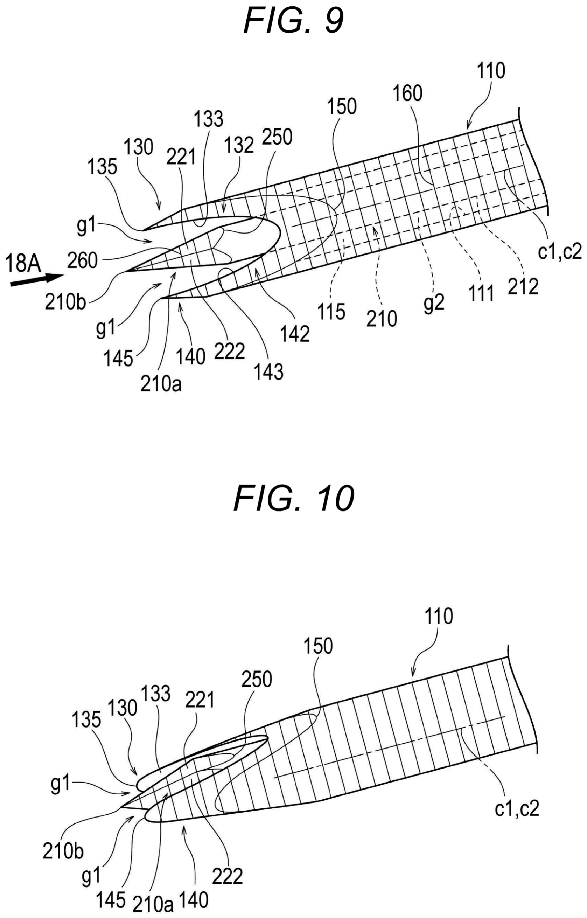

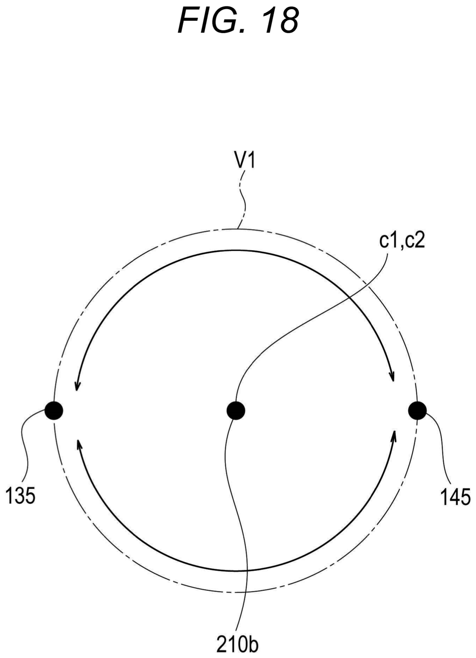

Fig. 18 illustrates a positional relationship between the first protruding portion 130 and the second protruding portion 140 with a position of the most distal end 210b of the inner needle 200 as a reference. Fig. 18 illustrates a plan view of the needles 100 and 200 viewed from the front side (plan view viewed from a direction indicated by an arrow 18A in Fig. 9) in the connected state of the puncture needle 10 in a simplified manner.

As illustrated in Fig. 18, the first protruding portion 130 and the second protruding portion 140 can be disposed at equal intervals in the circumferential direction along a virtual circle V1 centered on the most distal end 210b of the inner needle 200 in the connected state.

In the present embodiment, the puncture needle 10 is configured to cause the protruding portions 130 and 140 to rotate along a substantially perfect circular locus when the puncture needle 10 rotates with the most distal end 210b of the inner needle distal end 210a located at a position overlapping an axial center c2 of the inner needle 200 as a fulcrum.

In addition, two protruding portions 130 and 140 are formed on the outer needle 100. For that reason, in a plan view illustrated in Fig. 18, the protruding portions 130 and 140 are disposed at intervals of 180° from each other on the virtual circle V1 so as to face each other across the most distal end 210b of the inner needle distal end 210a.

As illustrated in Figs. 6 and 7, a certain range on the proximal end side from a most distal end 135 of the first protruding portion 130 and a certain range on the proximal end side from a most distal end 145 of the second protruding portion 140 are inclined toward an axial center c1 side of the outer needle main body 110 toward the distal end side from the proximal end side of the outer needle main body 110.

Specifically, the vicinity of the distal end of the side surface 131 located on the outer surface of the first protruding portion 130 has a tapered shape inclined substantially linearly toward the distal end side and the axial center c1 side in the side view (or front view) illustrated in Fig. 6. Similarly, the vicinity of the distal end of the side surface 141 located on the outer surface of the second protruding portion 140 has a tapered shape inclined substantially linearly toward the distal end side and the axial center c1 side in the side view (or front view) illustrated in Fig. 6. For that reason, in the outer needle main body 110, the outer diameter at each of the most distal ends 135 and 145 is the smallest as compared with other portions of the outer needle main body 110. Note that an inclination angle at which the vicinity of the distal end of each of the side surfaces 131 and 141 is inclined with respect to the axial center c1 can be, for example, 5° to 85°.

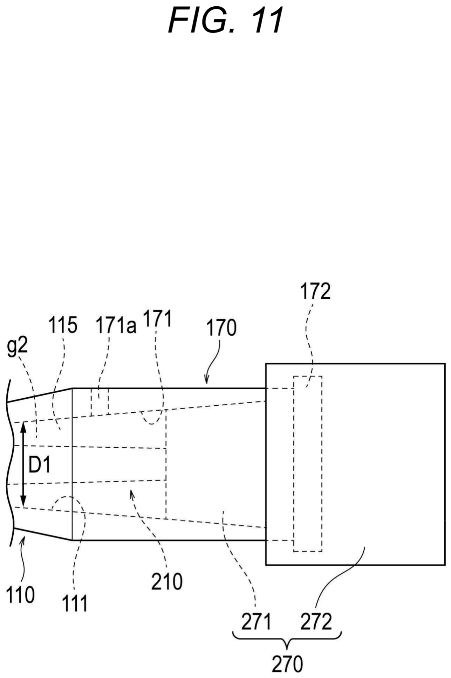

The outer needle 100 has a hollow structure in which the inner cavity 115 of the outer needle main body 110 is gradually larger at a position closer to the proximal end side from the distal end side. That is, the outer needle main body 110 is configured to cause a diameter D1 of the inner cavity 115 illustrated in Fig. 11 to be gradually larger at a position closer to the proximal end side.

A proximal end opening portion (not illustrated) of the outer needle 100 communicates with an insertion portion 171 of the outer needle hub 170.

As illustrated in Figs. 9 and 11, in the puncture needle 10 in the connected state, a gap portion g2 through which liquid can flow is formed between an outer surface 212 of the inner needle main body 210 and an inner surface 111 of the outer needle main body 110.

The gap portion g2 communicates with a gap g1 on the distal end side of the outer needle main body 110. For that reason, when the distal end of each of the needles 100 and 200 is inserted into the blood vessel B, the puncture needle 10 causes blood to flow into the gap portion g2 from the distal end side of the outer needle main body 110 through the gap g1. In addition, the puncture needle 10 can guide the blood flowing into the proximal end side through the gap portion g2 into the insertion portion 171 (see Fig. 11) of the outer needle hub 170 located on the proximal end side of the outer needle main body 110.

As illustrated in Figs. 6, 7, and 8, the outer needle 100 includes a first uneven portion 160 that enhances echo visibility and is formed in a predetermined range from the most distal end 135 of the first protruding portion 130 and the most distal end 145 of the second protruding portion 140 toward the proximal end side.

The first uneven portion 160 can include, for example, a groove portion extending orthogonal to the axial center c1 of the outer needle main body 110. However, the specific shape and structure of the first uneven portion 160 are not limited as long as a function of enhancing echo visibility is exerted. For example, the first uneven portion 160 can include a circular or rectangular groove portion, a groove portion designed in a spiral shape, a blasted portion including a fine uneven portion, a groove portion extending in a pattern of a colored spiral, and an uneven portion formed by arbitrarily combining these groove portions.

In the puncture needle 10, the hardness of the outer needle main body 110 is larger than the hardness of the inner needle main body 210. The magnitude relationship of the hardness can be defined, for example, on the basis of the Vickers hardness (ISO6507-1:2018) in the case of a metal material, and can be defined based on the Rockwell hardness (ISO2039-2:1987) in the case of a resin material. A material constituting the outer needle main body 110 is not particularly limited, and for example, the outer needle main body 110 can include a metal or a hard resin material. As the metal material constituting the outer needle main body 110, for example, it is possible to use stainless steel, aluminum or an aluminum alloy, titanium or a titanium alloy, or the like. On the other hand, as the resin material constituting the outer needle main body 110, for example, it is possible to use a thermoplastic resin such as polyethylene, polypropylene, polymethyl methacrylate, polycarbonate, polyamide, polyethylene terephthalate, or polyether ether ketone, and further, a mixture thereof may be used. In addition, as the resin material constituting the outer needle main body 110, a fluororesin may be used such as ECTFE (trifluorochloroethylene-ethylene copolymer resin), ETFE (tetrafluoroethylen-ethylene copolymer resin), FEP (tetrafluoroethylene-hexafluoropropylene copolymer resin), PCTFE (trifluorochloroethylene resin), PFA (tetrafluoroethylene-perfluoroalkyl vinyl ether copolymer resin), PTFE (tetrafluoroethylene resin), PVDF (vinylidene fluoride resin), or PVF (vinyl fluoride resin).

As illustrated in Fig. 1, the outer needle hub 170 includes the insertion portion 171, and a proximal end portion 172 disposed on the proximal end side of the insertion portion 171.

As illustrated in Figs. 2 and 11, in the puncture needle 10 in the connected state, a first connector portion 271 of the inner needle hub 270 is inserted into the insertion portion 171 of the outer needle hub 170. In addition, the puncture needle 10 is configured to cause the proximal end portion 172 of the outer needle hub 170 to be capable of maintaining a mechanical connection state with the second connector portion 272 of the inner needle hub 270 in the connected state.

As illustrated in Figs. 1, 2, and 11, the insertion portion 171 of the outer needle hub 170 is configured to cause a cross-sectional area to be larger at a position closer to the proximal end side. The puncture needle 10 is configured to cause the outer peripheral surface of the first connector portion 271 to come in contact with the inner peripheral surface of the insertion portion 171 in a state in which the first connector portion 271 of the inner needle hub 270 is inserted into the insertion portion 171 of the outer needle hub 170. For that reason, the puncture needle 10 can firmly maintain the connection between the outer needle hub 170 and the inner needle hub 270 in the connected state. In particular, in the puncture needle 10 in the connected state, the outer peripheral surface at a distal end position of the inner needle hub 270 (the position of the inner needle hub 270 on the inner needle main body 210 side) comes in contact with the inner peripheral surface of the insertion portion 171 of the outer needle hub 170, and thus, it is possible to more reliably prevent movement of the inner needle main body 210 with respect to the outer needle main body 110 when puncturing the puncture target site with the puncture needle 10 in the connected state.

In addition, the outer peripheral surface of the proximal end portion 172 of the outer needle hub 170 and the inner peripheral surface of the second connector portion 272 of the inner needle hub 270 can be provided with a structure for maintaining mechanical connection therebetween. For example, in the puncture needle 10, a male screw portion (or a female screw portion) can be provided on the outer peripheral surface of the proximal end portion 172 of the outer needle hub 170, and on the other hand, a female screw portion (or a male screw portion) can be provided on the inner peripheral surface of the second connector portion 272 of the inner needle hub 270. In the puncture needle 10, the proximal end portion 172 of the outer needle hub 170 and the second connector portion 272 of the inner needle hub 270 are provided with a mechanism for maintaining the connection therebetween as described above, so that the needles 100 and 200 can be integrally rotated together accompanied by each other when an operation of relatively rotating one of the outer needle 100 and the inner needle 200 with respect to the other is performed in the connected state.

Note that, in a case where the mechanism for maintaining the connection as described above is provided in each of the hubs 170 and 270 of the puncture needle 10, the mechanism can be configured to be capable of maintaining a positional relationship between the inner needle 200 and the outer needle 100 in which the gap g1 is formed between an inclined surface 220 of the inner needle 200 and each of the protruding portions 130 and 140 as illustrated in Figs. 2, 9, and 10 in a state in which the hubs 170 and 270 are connected together. In addition, the mechanism for maintaining the connection provided in each of the hubs 170 and 270 is not limited to a mechanism using a screw, and for example, can include a Luer taper configured to be able to maintain the connected state by fitting, a latch mechanism for maintaining the connected state by catching on a hole portion, or the like.

The outer needle hub 170 can include, for example, a transparent (including colorless transparent and translucent) hard resin. In the puncture needle 10, the outer needle hub 170 includes a transparent hard resin, whereby the operator can easily visually confirm the blood guided into the insertion portion 171 of the outer needle hub 170 through the gap portion g2. However, a material or the like constituting the outer needle hub 170 is not particularly limited, and for example, the outer needle hub 170 can include a colored hard resin, metal, or the like.

### <Inner Needle 200>

As illustrated in Figs. 1 and 2, the inner needle 200 includes the inner needle main body 210 having the inner needle distal end 210a that is sharp and the inner needle hub 270 connected to the proximal end side of the inner needle main body 210.

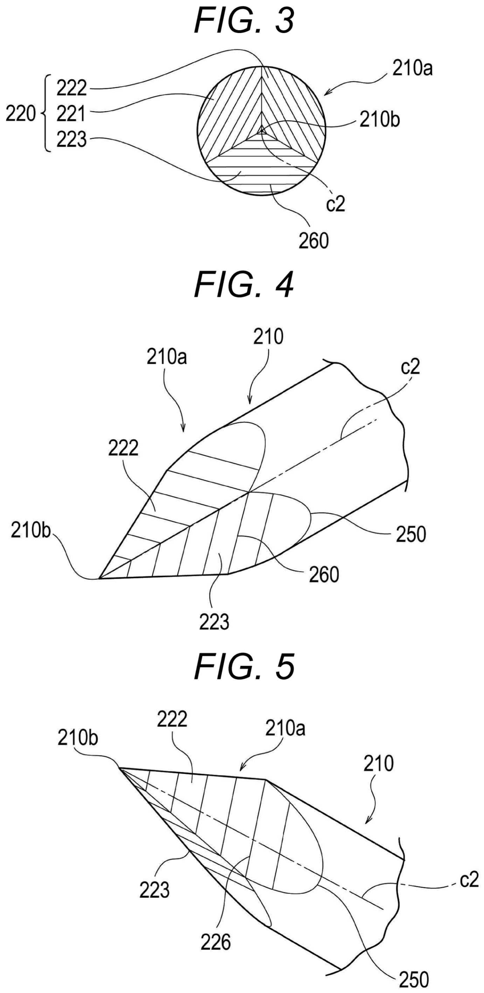

As illustrated in Figs. 3, 4, and 5, the inner needle distal end 210a of the inner needle main body 210 includes the inclined surface 220.

The inner needle main body 210 of the puncture needle 10 includes three inclined surfaces 221, 222, and 223. Hereinafter, the inclined surface 221 is referred to as a "first inclined surface", the inclined surface 222 is referred to as a "second inclined surface", and the inclined surface 223 is referred to as a "third inclined surface". Note that the generic name of the inclined surfaces 221, 222, and 223 is simply referred to as the "inclined surface 220".

As illustrated in Figs. 3, 4, and 5, each of the inclined surfaces 221, 222, and 223 is inclined from a predetermined position of the inner needle distal end 210a toward the distal end side and the axial center c2 side of the inner needle main body 210. The inclined surfaces 220 merge together so that an apex is formed at the most distal end 210b of the inner needle distal end 210a. The most distal end 210b forms a fulcrum when rotation operation of the puncture needle 10 is performed (see Figs. 16, 17, and 18). An inclination angle at which each of the inclined surfaces 221, 222, and 223 is inclined with respect to the axial center c2 of the inner needle main body 210 can be, for example, 5° to 85°.

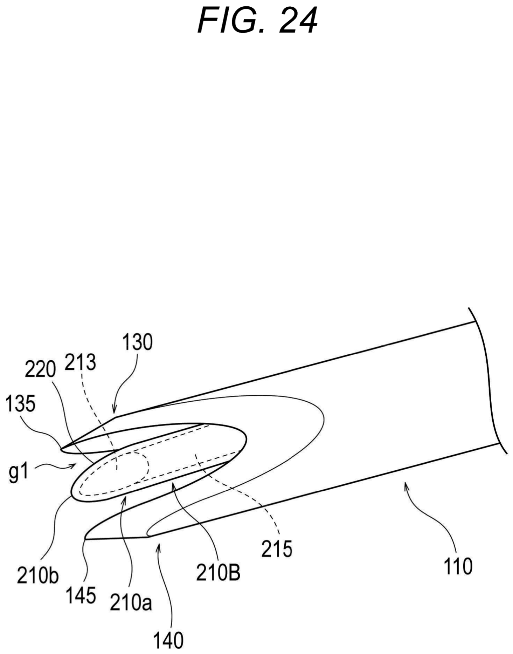

The inner needle main body 210 of the inner needle 200 has a solid structure. That is, an inner cavity through which a liquid such as blood can flow is not formed inside the inner needle 200. Note that, in the present embodiment, the inner needle 200 including the three inclined surfaces 221, 222, and 223 is exemplified, but the number of the inclined surfaces 220 included in the inner needle 200 only needs to be one or more. In Modification 3 described later, an inner needle including a hollow needle including one inclined surface 220 is exemplified (see Fig. 24).

As described above, the puncture needle 10 can be configured to cause the hardness of the outer needle main body 110 to be larger than the hardness of the inner needle main body 210. A material constituting the inner needle main body 210 is not particularly limited, and for example, in a case where SUS301 that is stainless steel is used as a constituent material of the outer needle main body 110, the inner needle main body 210 can include stainless steel such as SUS304, SUS304L, SUS305, SUS310S, SUS316, SUS316L, or SUS430.

Note that a method of providing a magnitude relationship of the hardness between the outer needle main body 110 and the inner needle main body 210 is not limited to a method using different materials as respective constituent materials. For example, it is also possible to provide a magnitude relationship of the hardness by adjusting surface treatment, residual compressive stress, and the like while forming the outer needle main body 110 and the inner needle main body 210 with the same material.

As illustrated in Figs. 3, 4, and 5, the inner needle 200 includes a second uneven portion 260 that enhances echo visibility and is formed between the most distal end 210b of the inner needle distal end 210a and a proximal end 250 of the inclined surface 220.

The second uneven portion 260 can include, for example, a groove portion extending along the inclined surface 220. However, the specific shape and structure of the second uneven portion 260 are not limited as long as a function of enhancing echo visibility is exerted.

For example, the second uneven portion 260 can include a circular or rectangular groove portion, a groove portion designed in a spiral shape, a blasted portion including a fine uneven portion, a groove portion extending in a pattern of a colored spiral, and an uneven portion formed by any combination of these groove portions.

As illustrated in Figs. 1, 2, and 11, the inner needle hub 270 includes the first connector portion 271, and the second connector portion 272 disposed on the proximal end side of the first connector portion 271.

As described above, the first connector portion 271 is inserted into the insertion portion 171 of the outer needle hub 170 in the connected state. In addition, the first connector portion 271 is configured to cause the outer peripheral surface of the first connector portion 271 to come in contact with the inner peripheral surface of the insertion portion 171 in the connected state. In addition, the second connector portion 272 can be configured to have a mechanism (for example, a screw portion) configured to be capable of maintaining mechanical connection with the proximal end portion 172 of the outer needle hub 170 in the connected state. A material or the like constituting the inner needle hub 270 is not particularly limited, and for example, the inner needle hub 270 can include a hard resin, metal, or the like.

As illustrated in Fig. 11, a hole portion 171a communicating with the insertion portion 171 of the outer needle hub 170 and the outside of the outer needle hub 170 can be formed at a predetermined position of the outer needle hub 170. Since the hole portion 171a is formed in the outer needle hub 170, the puncture needle 10 can prevent the insertion portion 171 of the outer needle hub 170 from being in a sealed state in a state in which the inner needle hub 270 and the outer needle hub 170 are connected together. As a result, when the puncture needle 10 is inserted into the blood vessel B in a state in which the inner needle hub 270 and the outer needle hub 170 are connected together, the puncture needle 10 can smoothly guide blood into the insertion portion 171 of the outer needle hub 170 through the gap portion g2. The operator can easily visually confirm the blood flowing into the insertion portion 171 from the outside of the outer needle hub 170.

Note that, in the present embodiment, an example has been described in which the hole portion 171a for ensuring a communication state between the inside of the insertion portion 171 and the outside of the outer needle hub 170 is provided in the outer needle hub 170; however, for example, it is also possible to form a hole portion having a function similar to that of the hole portion 171a in the inner needle hub 270. In a case where the hole portion is provided in the inner needle hub 270, the hole portion can be formed to extend from the distal end side to the proximal end side of the first connector portion 271 of the inner needle hub 270 so that the inside of the insertion portion 171 and the proximal end portion 172 of the outer needle hub 170 can communicate with each other in a state in which the outer needle hub 170 and the inner needle hub 270 are connected together.

The inner needle main body 210 can be formed to cause the outer diameter to be gradually smaller at a position closer to the proximal end side, for example (see Figs. 1 and 11). The inner needle main body 210 is configured as described above, whereby a cross-sectional area of the gap portion g2 can be made larger in the vicinity of the proximal end of the inner needle main body 210. For that reason, the operator can visually confirm the blood flowing into the gap portion g2 more easily when puncturing the blood vessel B with the puncture needle 10.

Here, Figs. 2, 9, and 10 illustrate the outer needle 100 and the inner needle 200 in the connected state.

In the puncture needle 10, when the outer needle 100 and the inner needle 200 are to be connected together, the inner needle main body 210 can be inserted into the inner cavity 115 of the outer needle main body 110 through a proximal end opening portion (not illustrated) formed at the proximal end of the outer needle hub 170. In addition, in the puncture needle 10, it is possible to connect the outer needle 100 and the inner needle 200 together by mechanically connecting the hubs 170 and 270 together while inserting the inner needle main body 210 into the inner cavity 115 of the outer needle main body 110.

As illustrated in Fig. 2, the puncture needle 10 of the present embodiment is configured to cause the axial center c1 of the outer needle 100 and the axial center c2 of the inner needle 200 to be coaxially disposed in the connected state.

As illustrated in Figs. 9 and 10, the puncture needle 10 is configured to cause the most distal end 210b of the inner needle distal end 210a to be located on the distal end side from the most distal end 135 of the first protruding portion 130 and the most distal end 145 of the second protruding portion 140 in the connected state. In addition, in the puncture needle 10 in the connected state, the inclined surface 220 of the inner needle distal end 210a is disposed to form the gap g1 between an inner surface 133 of the first protruding portion 130 and an inner surface 143 of the second protruding portion 140.

The puncture needle 10 can exert the following action and effect in the connected state.

Figs. 16 and 17 schematically illustrate a state when puncture is performed while the puncture needle 10 in the connected state is directed toward a predetermined puncture target site SA side.

As illustrated in Fig. 16, the operator brings the distal end side of the puncture needle 10 in the connected state close to the puncture target site SA. When the operator brings the puncture needle 10 close to the puncture target site SA, the most distal end 210b of the inner needle distal end 210a, located on the distal end side from the most distal ends 135 and 145 of the respective protruding portions 130 and 140, first comes into contact with the puncture target site SA. The operator rotates the entire puncture needle 10 clockwise and/or counterclockwise in a state in which the most distal end 210b of the inner needle distal end 210a is brought into contact with the puncture target site SA. The puncture needle 10 rotates with the most distal end 210b of the inner needle distal end 210a as a fulcrum such that the most distal ends 135 and 145 of the respective protruding portions 130 and 140 draw a perfect circle as illustrated in Fig. 18. The puncture needle 10 can form the perforation H in such a manner as to crush the puncture target site SA by the most distal ends 135 and 145 of the respective protruding portions 130 and 140.

In addition, in the puncture needle 10 in the connected state, the gap g1 is formed between the inclined surface 220 of the inner needle distal end 210a and the inner surfaces 133 and 143 of the respective protruding portions 130 and 140. For that reason, when forming the perforation H as described above, the puncture needle 10 can move the outer needle 100 and the inner needle 200 toward a puncture direction while releasing a part of the puncture target site SA in the gap g1. For that reason, the puncture needle 10 can smoothly form the perforation H toward a predetermined direction by rotating the puncture needle 10 while firmly bringing the most distal ends 135 and 145 of the respective protruding portions 130 and 140 and the most distal end 210b of the inner needle 200 into contact with the puncture target site SA.

The puncture needle 10 can be used, for example, to form the perforation H in the calcified lesion L illustrated in Figs. 19 and 20.

As illustrated in Fig. 19, in a case where the calcified lesion L is formed on the skin tissue S side of the blood vessel wall Bw of the blood vessel B, the perforation H may not be easily formed in the hard calcified lesion L. In such a case, by using the puncture needle 10 and performing the operation of rotating the puncture needle 10 as described above, it is possible to easily form the perforation H in such a manner as to crush the puncture target site SA by the most distal ends 135 and 145 of the respective protruding portions 130 and 140.

As illustrated in Fig. 20, after forming the perforation H in the calcified lesion L, the operator removes the inner needle 200 from the outer needle 100. The operator can insert various medical instruments (such as a guide wire) into the blood vessel B through the inner cavity 115 of the outer needle main body 110 and deliver the medical instruments to a blood vessel (for example, a stenosed part of the blood vessel) to be treated.

The operator can form the perforation H having a relatively large diameter by performing a rotation operation with the most distal end 210b of the inner needle 200 as a fulcrum in the procedure using the puncture needle 10 as described above. For that reason, the operator can also easily insert a catheter or the like having a relatively large outer diameter into the blood vessel B through a guide wire remaining in the blood vessel B by inserting the guide wire into the blood vessel B through the perforation H and then removing the outer needle 100 from the body.

In the puncture needle 10, the cross-sectional area of the insertion portion 171 of the outer needle hub 170 is larger at a position closer to the proximal end side. For that reason, when inserting a medical instrument or a medical device from the outer needle hub 170 in a state in which the inner needle 200 is removed from the outer needle 100, the operator can smoothly introduce the medical instrument or the medical device into the insertion portion 171. In addition, in the puncture needle 10, a stepped portion that hinders smooth movement of the medical instrument or the medical device is not formed in the inner cavity 115 of the outer needle 100, the insertion portion 171 of the outer needle hub 170, and the like. For that reason, the operator can smoothly move the medical instrument or the medical device inserted into the inner cavity 115 of the outer needle 100 and the insertion portion 171 of the outer needle hub 170.

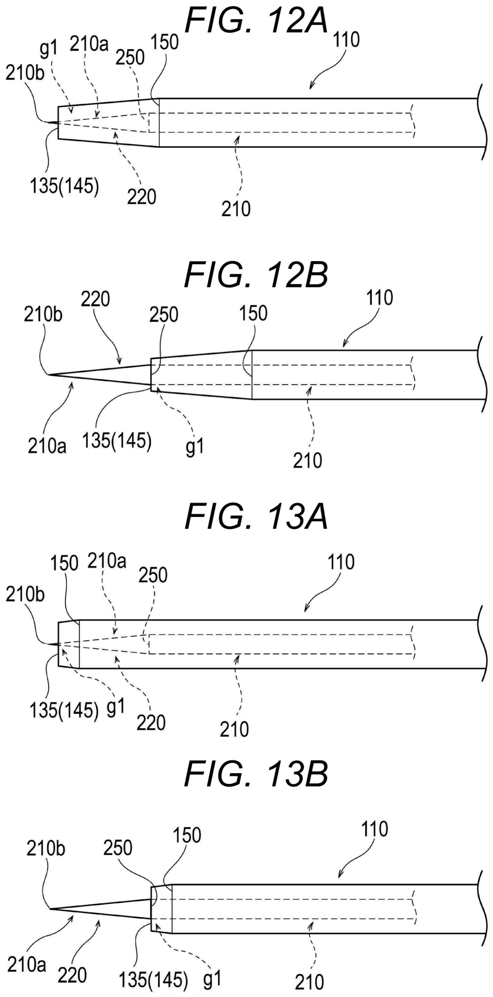

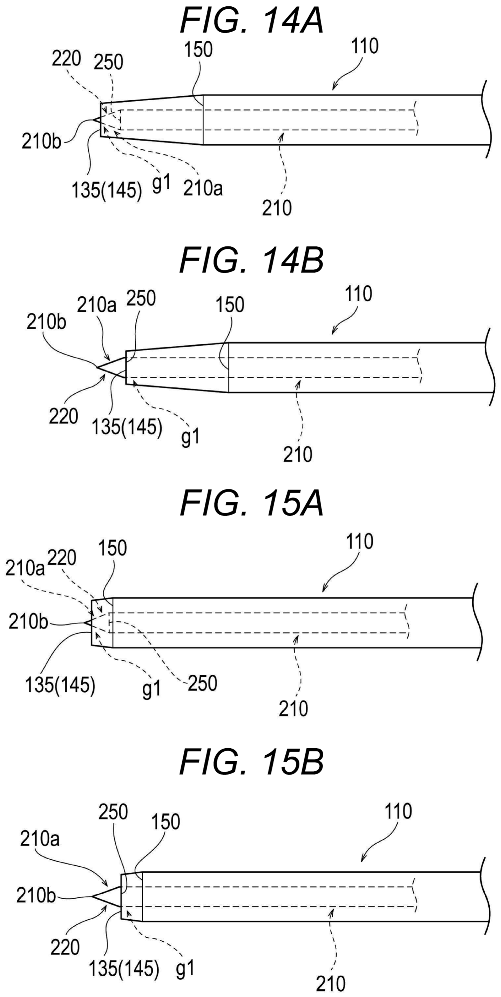

Next, with reference to Figs. 12A to 15B, a description will be given of arrangement examples of the most distal ends 135 and 145 of the respective protruding portions 130 and 140 and the most distal end 210b of the inner needle distal end 210a. Note that, in Figs. 12 to 15, schematic drawings are illustrated for the purpose of explaining an outline of the arrangement examples.

As described above, the puncture needle 10 can be configured to cause the most distal end 210b of the inner needle distal end 210a to be disposed on the distal end side from the most distal ends 135 and 145 of the respective protruding portions 130 and 140 in the connected state in order to enable smooth formation of the perforation H in the puncture target site SA.

On the premise that the puncture needle 10 is configured as described above, it is further preferable that the most distal end 135 of the first protruding portion 130 and the most distal end 145 of the second protruding portion 140 are disposed on the distal end side from the proximal end 250 of the inclined surface 220 in the connected state (see Figs. 9 and 10). This is for the following reason.

In the puncture needle 10, the most distal ends 135 and 145 of the respective protruding portions 130 and 140 are disposed on the distal end side from the proximal end 250 of the inclined surface 220 in the connected state, so that it is possible to bring the most distal ends 135 and 145 of the respective protruding portions 130 and 140 into contact with the puncture target site SA following the most distal end 210b of the inner needle distal end 210a when the puncture needle 10 is brought into contact with the puncture target site SA from the distal end side. As a result, the puncture needle 10 can smoothly insert the most distal ends 135 and 145 of the respective protruding portions 130 and 140 located on the proximal end side of the inclined surface 220 into the puncture target site SA before the proximal end 250 of the inclined surface 220 reaches the puncture target site SA after inserting the inclined surface 220 from the most distal end 210b of the inner needle distal end 210a.

For example, in a case where the puncture needle 10 is used for puncturing the blood vessel B illustrated in Figs. 19 and 20, an axial length (linear distance in the axial direction of the outer needle 100 in a plan view from each of the most distal ends 135 and 145 to the proximal end 150 of each of the blade surfaces 132 and 142 illustrated in Figs. 12A to 15B) of each of the protruding portions 130 and 140 of the outer needle 100 can be formed to be 0.5 mm to 10 mm. In addition, in the case of configuring the outer needle 100 in this manner, an axial length (linear distance in the axial direction of the inner needle 200 in a plan view from the most distal end 210b to the proximal end 250 of the inclined surface 220) of the inclined surface 220 of the inner needle 200 in the axial direction can be formed to be 0.5 mm to 10 mm. In the case of considering the axial length of each of the protruding portions 130 and 140 and the axial length of the inclined surface 220 of the inner needle 200 exemplified above (in the case of being configured by combining a maximum value and a minimum value of the axial lengths), for example, the arrangement examples illustrated in Figs. 12A to 15B can be adopted.

In the example illustrated in Fig. 12A, each of the protruding portions 130 and 140 is configured to have an axial length of 10 mm. In addition, the inclined surface 220 of the inner needle 200 is configured to have an axial length of 10 mm. In the arrangement example illustrated in Fig. 12A, a protruding length of the inclined surface 220 of the inner needle 200 from the outer needle 100 is 0.1 mm.

In the example illustrated in Fig. 12B, each of the protruding portions 130 and 140 is configured to have an axial length of 10 mm. In addition, the inclined surface 220 of the inner needle 200 is configured to have an axial length of 10 mm. In the arrangement example illustrated in Fig. 12B, the protruding length of the inclined surface 220 of the inner needle 200 from the outer needle 100 is 9.9 mm.

In the example illustrated in Fig. 13A, each of the protruding portions 130 and 140 is configured to have an axial length of 0.5 mm. In addition, the inclined surface 220 of the inner needle 200 is configured to have an axial length of 10 mm. In the arrangement example illustrated in Fig. 13A, the protruding length of the inclined surface 220 of the inner needle 200 from the outer needle 100 is 0.1 mm.

In the example illustrated in Fig. 13B, each of the protruding portions 130 and 140 is configured to have an axial length of 0.5 mm. In addition, the inclined surface 220 of the inner needle 200 is configured to have an axial length of 10 mm. In the arrangement example illustrated in Fig. 13B, the protruding length of the inclined surface 220 of the inner needle 200 from the outer needle 100 is 9.9 mm.

In the example illustrated in Fig. 14A, each of the protruding portions 130 and 140 is configured to have an axial length of 10 mm. In addition, the inclined surface 220 of the inner needle 200 is configured to have an axial length of 0.5 mm. In the arrangement example illustrated in Fig. 14A, the protruding length of the inclined surface 220 of the inner needle 200 from the outer needle 100 is 0.1 mm.

In the example illustrated in Fig. 14B, each of the protruding portions 130 and 140 is configured to have an axial length of 10 mm. In addition, the inclined surface 220 of the inner needle 200 is configured to have an axial length of 0.5 mm. In the arrangement example illustrated in Fig. 14B, the protruding length of the inclined surface 220 of the inner needle 200 from the outer needle 100 is 0.4 mm.

In the example illustrated in Fig. 15A, each of the protruding portions 130 and 140 is configured to have an axial length of 0.5 mm. In addition, the inclined surface 220 of the inner needle 200 is configured to have an axial length of 0.5 mm. In the arrangement example illustrated in Fig. 15A, the protruding length of the inclined surface 220 of the inner needle 200 from the outer needle 100 is 0.1 mm.

In the example illustrated in Fig. 15B, each of the protruding portions 130 and 140 is configured to have an axial length of 0.5 mm. In addition, the inclined surface 220 of the inner needle 200 is configured to have an axial length of 0.5 mm. In the arrangement example illustrated in Fig. 15B, the protruding length of the inclined surface 220 of the inner needle 200 from the outer needle 100 is 0.4 mm.

### <Action and Effect>

As described above, the puncture needle 10 according to the present embodiment includes the outer needle 100, and the inner needle 200 configured to be insertable into the outer needle 100. The outer needle 100 includes the outer needle main body 110 having the inner cavity 115, and the outer needle hub 170 disposed on the proximal end side of the outer needle main body 110. The inner needle 200 includes the inner needle main body 210 having the inner needle distal end 210a that is sharp, and the inner needle hub 270 connected to the proximal end side of the inner needle main body 210. The distal end 110a of the outer needle main body 110 includes the first protruding portion 130 and the second protruding portion 140 having the blade surfaces 132 and 142 on the side surfaces 131 and 141. The inner needle distal end 210a includes the inclined surface 220. In the connected state in which the inner needle main body 210 is inserted into the outer needle main body 110, and the inner needle hub 270 and the outer needle hub 170 are connected together, the most distal end 210b of the inner needle distal end 210a is located on the distal end side from the most distal end 135 of the first protruding portion 130 and the most distal end 145 of the second protruding portion 140, and the inner needle distal end 210a is disposed such that the inclined surface 220 forms the gap g1 between the inner surface 133 of the first protruding portion 130 and the inner surface 143 of the second protruding portion 140.

In the connected state in which the inner needle 200 and the outer needle 100 are connected together, the puncture needle 10 configured as described above is disposed in a state in which the most distal end 210b of the inner needle distal end 210a of the inner needle 200 and the most distal ends 135 and 145 of the respective protruding portions 130 and 140 of the outer needle 100 are exposed at the distal end of the puncture needle 10. In addition, in the connected state, the most distal end 210b of the inner needle distal end 210a is located on the distal end side from the most distal end 135 of the first protruding portion 130 of the outer needle 100 and the most distal end 145 of the second protruding portion 140 of the outer needle 100.

When puncturing the puncture target site SA with the puncture needle 10, the operator rotates the puncture needle 10 with the most distal end 210b of the inner needle distal end 210a as a fulcrum. When the operator rotates the puncture needle 10, the most distal ends 135 and 145 of the respective protruding portions 130 and 140 of the outer needle 100 start to hit the puncture target site SA in a state in which the most distal end 210b of the inner needle distal end 210a is in contact with the puncture target site SA. The operator rotates the puncture needle 10 to rotate the protruding portions 130 and 140 of the outer needle 100 located around the inner needle 200, thereby being able to form the perforation H in such a manner as to crush the puncture target site SA by the protruding portions 130 and 140 of the outer needle 100. For that reason, for example, even in a case where the puncture target site SA includes the calcified lesion L or the like that has hardened, the operator can smoothly form the perforation H by the puncture needle 10. In addition, in the puncture needle 10 in the connected state, the inclined surface 220 of the inner needle distal end 210a is disposed to form the gap g1 between the inner surface 133 of the first protruding portion 130 of the outer needle 100 and the inner surface 143 of the second protruding portion 140 of the outer needle 100. For that reason, when an operation is performed of rotating the puncture needle 10 with the most distal end 210b of the inner needle distal end 210a as a fulcrum, it is possible to move the outer needle 100 and the inner needle 200 toward the puncture direction while releasing a part of the puncture target site SA (for example, a part of a biological tissue or a part of the calcified lesion L) into the gap g1. For that reason, the operator can smoothly form the perforation H toward a predetermined direction by rotating the puncture needle 10 while bringing the most distal ends 135 and 145 of the respective protruding portions 130 and 140 of the outer needle 100 and the most distal end 210b of the inner needle distal end 210a into contact with the puncture target site SA.

In addition, the first protruding portion 130 and the second protruding portion 140 are disposed at equal intervals in the circumferential direction along the virtual circle V1 centered on the most distal end 210b of the inner needle distal end 210a in the connected state.

According to the puncture needle 10 configured as described above, when performing puncture with the puncture needle 10, the operator can move the most distal ends 135 and 145 of the respective protruding portions 130 and 140 of the outer needle 100 along the virtual circle V1 centered on the most distal end 210b of the inner needle distal end 210a by rotating the puncture needle 10. In addition, the operator can efficiently form the perforation H in the puncture target site SA by the most distal ends 135 and 145 of the respective protruding portions 130 and 140 by performing an operation of rotating the puncture needle 10 clockwise and an operation (return operation) of rotating the puncture needle 180° counterclockwise.

In the connected state, the most distal end 135 of the first protruding portion 130 and the most distal end 145 of the second protruding portion 140 are disposed on the distal end side from the proximal end 250 of the inclined surface 220 of the inner needle distal end 210a.

According to the puncture needle 10 configured as described above, since the most distal ends 135 and 145 of the respective protruding portions 130 and 140 are disposed on the distal end side from the proximal end 250 of the inclined surface 220 in the connected state, it is possible to bring the most distal ends 135 and 145 of the respective protruding portions 130 and 140 into contact with the puncture target site SA following the most distal end 210b of the inner needle distal end 210a when the puncture needle 10 is moved from the distal end side toward the puncture target site SA. Thus, the puncture needle 10 can smoothly insert the most distal ends 135 and 145 of the respective protruding portions 130 and 140 located on the proximal end side of the inclined surface 220 into the puncture target site SA before the proximal end 250 of the inclined surface 220 reaches the puncture target site SA when the inclined surface 220 is inserted into the puncture target site SA from the most distal end 210b of the inner needle distal end 210a. As a result, the puncture needle 10 can improve puncture efficiency by each of the protruding portions 130 and 140 of the outer needle 100.

In addition, a certain range on the proximal end side from the most distal end 135 of the first protruding portion 130 and a certain range on the proximal end side from the most distal end 145 of the second protruding portion 140 are inclined toward the axial center c1 side of the outer needle main body 110 toward the distal end side from the proximal end side of the outer needle main body 110.

According to the puncture needle 10 configured as described above, a portion located on the most distal end 135 side of the first protruding portion 130 and a portion located on the most distal end 145 side of the second protruding portion 140 each have a sharp outer surface inclined toward the axial center c1 side of the outer needle main body 110.

For that reason, the puncture needle 10 is improved in puncture performance and crushing performance of the protruding portions 130 and 140.

In addition, the hardness of the outer needle main body 110 is larger than the hardness of the inner needle main body 210.

According to the puncture needle 10 configured as described above, in a state in which the protruding portions 130 and 140 of the outer needle 100 hit the blood vessel B that has hardened due to the calcified lesion L or the like and start to crush the blood vessel B when the puncture needle 10 is rotated with the inner needle distal end 210a as a fulcrum, it is possible to prevent the most distal ends 135 and 145 of the respective protruding portions 130 and 140 of the outer needle 100 from being easily deformed due to contact with the calcified lesion L or the like. For that reason, when crushing the calcified lesion L or the like by the protruding portions 130 and 140, the puncture needle 10 can maintain the shape of the most distal ends 135 and 145 of the respective protruding portions 130 and 140 in a predetermined shape. As a result, the puncture needle 10 is further improved in the puncture performance and the crushing performance.

In addition, in the inner needle 200, the inner needle main body 210 has a solid structure, and the outer needle 100 has a hollow structure in which the inner cavity 115 of the outer needle main body 110 is gradually larger at a position closer to the proximal end side from the distal end side. In addition, in the connected state, the gap portion g2 through which liquid can flow is formed between the outer surface 212 of the inner needle main body 210 and the inner surface 111 of the outer needle main body 110.

According to the puncture needle 10 configured as described above, when the puncture needle 10 is inserted into the blood vessel B, the blood flowing through the blood vessel B flows into the gap portion g2. The operator can confirm that the puncture needle 10 has been inserted into the blood vessel B by visually confirming the blood flowing into the gap portion g2 through the outer needle hub 170. In addition, since the inner cavity 115 of the outer needle main body 110 of the outer needle 100 is gradually larger at a position closer to the proximal end side, the cross-sectional area of the gap portion g2 formed between the outer surface 212 of the inner needle main body 210 and the inner surface 111 of the outer needle main body 110 is also larger at a position closer to the proximal end side. For that reason, the operator can more easily visually confirm the flow of blood through the outer needle hub 170 in the vicinity of the proximal end of the outer needle main body 110.

In addition, the outer needle 100 includes the first uneven portion 160 that enhances echo visibility and is formed in a predetermined range from the most distal end 135 of the first protruding portion 130 and the most distal end 145 of the second protruding portion 140 toward the proximal end side. The inner needle 200 includes the second uneven portion 260 that enhances echo visibility and is formed between the most distal end 210b of the inner needle distal end 210a and the proximal end 250 of the inclined surface 220.

According to the puncture needle 10 configured as described above, the echo visibility in a certain range on the distal end side of each of the protruding portions 130 and 140 and the echo visibility in a certain range of the inner needle distal end 210a can be suitably enhanced by the first uneven portion 160 and the second uneven portion 260. In addition, the puncture needle 10 can efficiently enhance echo visibility in a range in which the first uneven portion 160 of the outer needle 100 and the second uneven portion 260 of the inner needle 200 coincide with each other in the circumferential direction in the connected state. For that reason, the operator can more accurately grasp positions of the protruding portions 130 and 140 and the inner needle distal end 210a under echo visual recognition. In addition, in the puncture needle 10 in the connected state, the first uneven portion 160 of the outer needle 100 and the second uneven portion 260 of the inner needle 200 increase a surface area of a region where echo visibility is enhanced on the distal end side of the puncture needle 10, so that a distal end position of the puncture needle 10 can be grasped more accurately.

Next, a description will be given of a puncture needle according to a modification of the embodiment described above. In the description of the modification, the description will be appropriately omitted of the action and effect of the member and the puncture needle already described. Furthermore, contents that are not particularly described in the modification can be the same as those in the embodiment described above.

### (Modification 1)

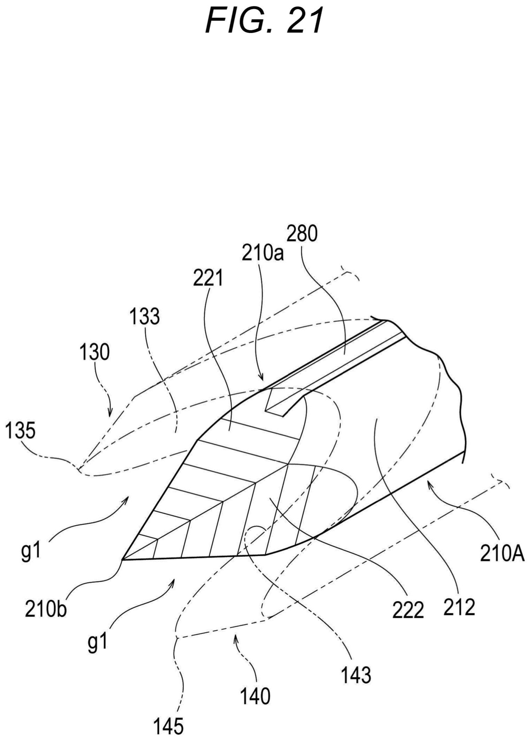

Fig. 21 is a perspective view illustrating the inner needle distal end 210a of an inner needle main body 210A according to Modification 1.

A recessed groove portion 280 extending along the axial direction of the inner needle main body 210A is formed in the outer surface 212 of the inner needle main body 210A.

The recessed groove portion 280 can be configured to be disposed between the first protruding portion 130 and the second protruding portion 140 in the connected state.

When puncturing the blood vessel B with the inner needle 200 in a procedure using the inner needle main body 210A, the operator can cause the blood flowing through the blood vessel B to flow into the recessed groove portion 280. The operator can visually confirm that the blood flowing along the recessed groove portion 280 has reached each of the hubs 170 and 270. By using an inner needle including the inner needle main body 210A according to Modification 1, the operator can more easily confirm the blood flashback when the puncture needle punctures the blood vessel B.

### (Modification 2)

Figs. 22 and 23 are perspective views illustrating the protruding portions 130, 140, and 180 of an outer needle main body 110A according to Modification 2.

The number of protruding portions included in the outer needle main body 110A is not particularly limited as long as it is two or more. In the present modification, the outer needle main body 110A is configured to include three protruding portions 130, 140, and 180 disposed at different positions around the axial center c1 of the outer needle main body 110A.

As with the other protruding portions 130 and 140, the protruding portion 180 includes a side surface 181 on which a blade surface 182 is formed and a most distal end 185.

Note that, in a case where the outer needle main body 110A according to Modification 2 is connected to the inner needle, inclined surfaces of the inner needle main body can be disposed so as to form the gap g1 between the inner surfaces 133, 143, and 183 of the respective protruding portions 130, 140, and 180.

### (Modification 3)

Fig. 24 illustrates the inner needle distal end 210a of an inner needle main body 210B according to Modification 3.

As illustrated in Fig. 24, the inner needle main body 210B can include, for example, a hollow needle including an inner cavity 215.

A distal end opening portion 213 communicating with the inner cavity 215 is formed at the distal end of the inner needle main body 210B including the hollow needle. In addition, one inclined surface 220 extending substantially parallel to an opening direction of the distal end opening portion 213 is formed at the inner needle distal end 210a of the inner needle main body 210B. As illustrated in the present modification, in a case where the inner needle main body 210B is configured to include one inclined surface 220, the puncture needle can be configured to cause the inclined surface 220 to form the gap g1 with the inner surface 133 or 143 of any of the plurality of protruding portions 130 and 140 in the connected state. That is, the puncture needle can be configured to cause the gap g1 to be formed between one or a plurality of inclined surfaces and any protruding portion among a plurality of protruding portions according to the number of the inclined surfaces 220 and the number of the protruding portions.

In the puncture needle, in a case where the inner needle main body 210B includes a hollow needle, for example, the inner needle hub 270 (see Fig. 11) can include a transparent (including colorless transparent and translucent) hard resin. In a case where the outer needle hub 170 includes a transparent material, and the inner needle hub 270 includes a transparent material, the operator can easily visually confirm the blood flowing into the inner cavity 215 of the inner needle main body 210B and further guided to the insertion portion 171 when the inner needle main body 210B is inserted into the blood vessel B, from the outside of each of the hubs 170 and 270.

### (Modification 4)

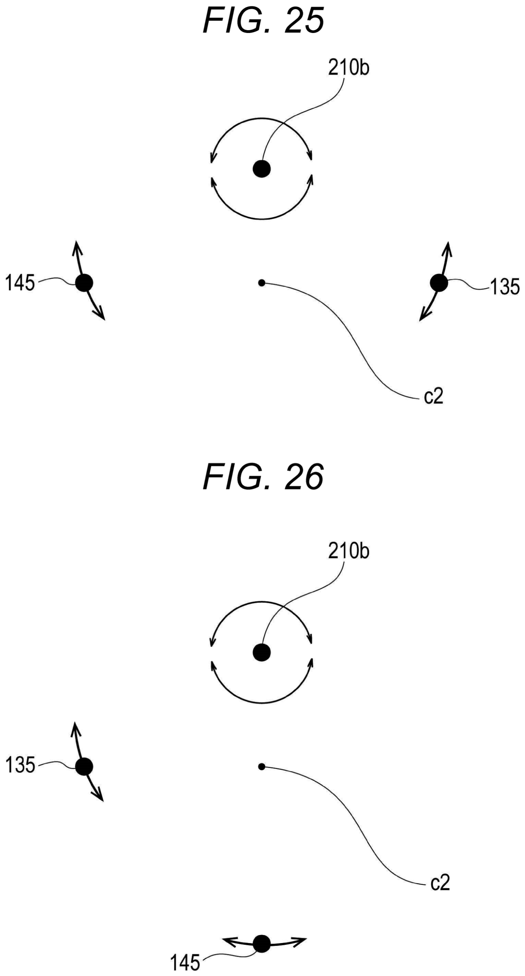

Fig. 25 is a diagram illustrating a puncture needle according to Modification 4. Fig. 25 is a front view corresponding to Fig. 18 of the embodiment described above.

In the connected state, the puncture needle is not particularly limited as to a position where the most distal end 210b of the inner needle distal end 210a is disposed, a position where the most distal end 135 of the first protruding portion 130 is disposed, and a position where the most distal end 145 of the second protruding portion 140 is disposed. For example, as illustrated in Fig. 25, in the puncture needle in the connected state, it is also possible to dispose the most distal end 210b of the inner needle distal end 210a at a position shifted from the axial center c2 of the inner needle main body 210. In a case where the most distal end 210b of the inner needle distal end 210a is disposed in this manner, the first protruding portion 130 and the second protruding portion 140 can be disposed at positions facing each other across the axial center c2 of the inner needle 200.

Even in a case where the most distal end 210b of the inner needle distal end 210a, the most distal end 135 of the first protruding portion 130, and the most distal end 145 of the second protruding portion 140 are disposed as described in the present modification, as long as the puncture needle is configured to cause the most distal end 210b of the inner needle distal end 210a to be disposed on the distal end side of the most distal ends 135 and 145 of the respective protruding portions 130 and 140, and cause the gap g1 to be formed between the most distal end 210b of the inner needle distal end 210a and the inner surfaces 133 and 143 of the protruding portions 130 and 140, in the connected state, it is possible to achieve smooth puncture work on the puncture target site SA.

Note that, even in a case where a position of the most distal end 210b of the inner needle distal end 210a is disposed at a position shifted from the axial center c2 of the inner needle 200 and intervals between the first protruding portion 130 and the second protruding portion 140 are not uniform as in Modification 5 illustrated in Fig. 26 (a case where the protruding portions are not disposed so as to face each other across the axial center c2 of the inner needle main body 210), the puncture needle can achieve smooth puncture work on the puncture target site SA as in Modification 4 described above.

Although the puncture needle according to the present invention has been described above through the plurality of embodiments and the plurality of modifications, the present invention is not limited only to the contents described in the specification and can be appropriately changed on the basis of the description of the claims.

For example, the configuration described in the embodiment and the configurations described in the modifications can be arbitrarily combined as long as the effect of the invention is exerted. As an example, the outer needle described in Modification 2 and the inner needle described in Modification 3 can be provided with a first uneven portion and a second uneven portion that enhances echo visibility.

The present application is based on Japanese Patent Application No. 2023-003575 filed on January 13, 2023, the disclosure content of which is incorporated herein by reference in its entirety.

### Reference Signs List

10 Puncture needle
100 Outer needle
110 Outer needle main body
110A Outer needle main body
110a Distal end of outer needle main body
111 Inner surface of outer needle main body
115 Inner cavity of outer needle main body
130 First protruding portion
131 Side surface of first protruding portion
132 Blade surface of first protruding portion
133 Inner surface of first protruding portion
135 Most distal end of first protruding portion
140 Second protruding portion
141 Side surface of second protruding portion
142 Blade surface of second protruding portion
143 Inner surface of second protruding portion
145 Most distal end of second protruding portion
150 Proximal end of blade surface
160 First uneven portion
170 Outer needle hub
171 Insertion portion of outer needle hub
172 Proximal end portion of outer needle hub
180 Protruding portion
200 Inner needle
210 Inner needle main body
210A Inner needle main body
210B Inner needle main body
210a Inner needle distal end
210b Most distal end of inner needle distal end
212 Outer surface of inner needle main body
213 Distal end opening portion
215 Inner cavity of inner needle main body
220 Inclined surface
221 First inclined surface
222 Second inclined surface
223 Third inclined surface
250 Proximal end of inclined surface
260 Second uneven portion
270 Inner needle hub
271 First connector portion
272 Second connector portion
280 Recessed groove portion
B Blood vessel
Bw Blood vessel wall
H Perforation
L Calcified lesion
S Skin tissue
SA Puncture target site
V1 Virtual circle
c1 Axial center of outer needle main body
c2 Axial center of inner needle main body
g1 Gap
g2 Gap portion

## Claims

1. A puncture needle comprising an outer needle, and an inner needle configured to be insertable into the outer needle, wherein:
the outer needle includes an outer needle main body having an inner cavity, and an outer needle hub disposed on a proximal end side of the outer needle main body;
the inner needle includes an inner needle main body having an inner needle distal end that is sharp, and an inner needle hub connected to the proximal end side of the inner needle main body;
a distal end of the outer needle main body includes a first protruding portion and a second protruding portion each having a blade surface on a side surface;
the inner needle distal end includes an inclined surface; and
in a connected state in which the inner needle main body is inserted into the outer needle main body and the inner needle hub and the outer needle hub are connected together, a most distal end of the inner needle distal end is located on a distal end side from a most distal end of the first protruding portion and a most distal end of the second protruding portion, and the inner needle distal end is disposed such that the inclined surface forms a gap with an inner surface of the first protruding portion and/or an inner surface of the second protruding portion.

2. The puncture needle according to claim 1, wherein the first protruding portion and the second protruding portion are disposed at equal intervals in a circumferential direction along a virtual circle centered on the most distal end of the inner needle distal end in the connected state.

3. The puncture needle according to claim 1, wherein the most distal end of the first protruding portion and the most distal end of the second protruding portion are disposed on the distal end side from a proximal end of the inclined surface in the connected state.

4. The puncture needle according to claim 1, wherein a certain range on the proximal end side from the most distal end of the first protruding portion and a certain range on the proximal end side from the most distal end of the second protruding portion are inclined toward an axial center side of the outer needle main body toward the distal end side from the proximal end side of the outer needle main body.

5. The puncture needle according to claim 1, wherein hardness of the outer needle main body is larger than hardness of the inner needle main body.

6. The puncture needle according to claim 1, wherein a recessed groove portion extending along an axial direction of the inner needle main body is formed on an outer surface of the inner needle main body.

7. The puncture needle according to claim 1, wherein:
the inner needle main body of the inner needle has a solid structure;
the outer needle has a hollow structure in which the inner cavity of the outer needle main body is gradually larger at a position closer to the proximal end side from the distal end side; and
a gap portion through which liquid is allowed to flow is formed between an outer surface of the inner needle main body and an inner surface of the outer needle main body in the connected state.

8. The puncture needle according to claim 1, wherein:
the outer needle includes a first uneven portion that enhances echo visibility and is formed in a predetermined range toward the proximal end side from the most distal end of the first protruding portion and the most distal end of the second protruding portion; and
the inner needle includes a second uneven portion that enhances echo visibility and is formed between the most distal end of the inner needle distal end and a proximal end of the inclined surface.
